# EUROPEAN PATENT SPECIFICATION

(11) **EP 3 266 773 B1**
(45) Date of publication and mention of the grant of the patent: **11.04.2018**
(21) Application number: 16177841.0
(22) Date of filing: 04.07.2016
(51) Int. Cl.: C07D 405/04

(54) **PROCESS FOR THE PREPARATION OF LAPATINIB DITOSYLATE MONOHYDRATE BY MEANS OF AN IMPROVED CRYSTALLIZATION PROCEDURE**
VERFAHREN ZUR HERSTELLUNG VON LAPATINIB DITOSYLAT MONOHYDRAT MITTELS EINES VERBESSERTEN KRISTALLISIERUNGSPROZESSES
PROCÉDÉ DE PRÉPARATION DE LAPATINIB DITOSYLATE MONOHYDRATE PAR UN PROCÉDÉ DE CRISTALLISATION AMÉLIORÉ

(43) Date of publication of application: 10.01.2018
(73) Proprietor: F.I.S.- Fabbrica Italiana Sintetici S.p.A., 36075 Montecchio Maggiore (VI) (IT)
(72) Inventor: Fontana, Francesco, 22030 Longone al Segrino (IT)
(74) Representative: Leganza, Alessandro

(56) References cited:
- WO-A1-02/02552
- WO-A1-2009/079541
- WO-A1-2011/116634
- WO-A2-2009/137714
- US-A1- 2015 260 696

## Description

### Technical Field

The present invention provides a process for the preparation of pharmaceutical active ingredient Lapatinib by means of an improved crystallization procedure.

### Background Art

Lapatinib is a pharmaceutical active ingredient used for the treatment of breast cancer and other solid tumours, in particular it is used for the treatment of patients with advanced or metastatic breast cancer whose tumours overexpress HER2 (ErbB2).

Lapatinib is available in the market under the name Tykerb® (US) or Tyverb® (EU).

According to the indication of the manufacture, Tykerb® or Tyverb® contains Lapatinib as ditosylate monohydrate salt of formula (I): 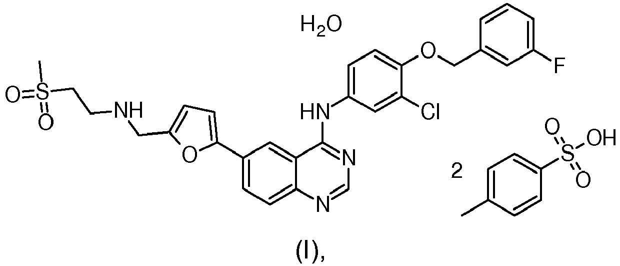 having the chemical name of N-{3-Chloro-4-[(3-fluorobenzyl)oxy]phenyl}-6-[5-({[2-(methylsulphonyl)ethyl]amino}methyl)furan-2-yl] quinazolin-4-amine bis(4-methylbenzenesulphonate) monohydrate, CAS RN 388082-78-8 and m.p. 250-256°C.

Several methods for the preparation of Lapatinb ditosylate monohydrate have been disclosed, in particular some of these processes for obtaining the final product have been carried out using as starting material one of the many disclosed polymorph forms of Lapatinb ditosylate anhydrous.

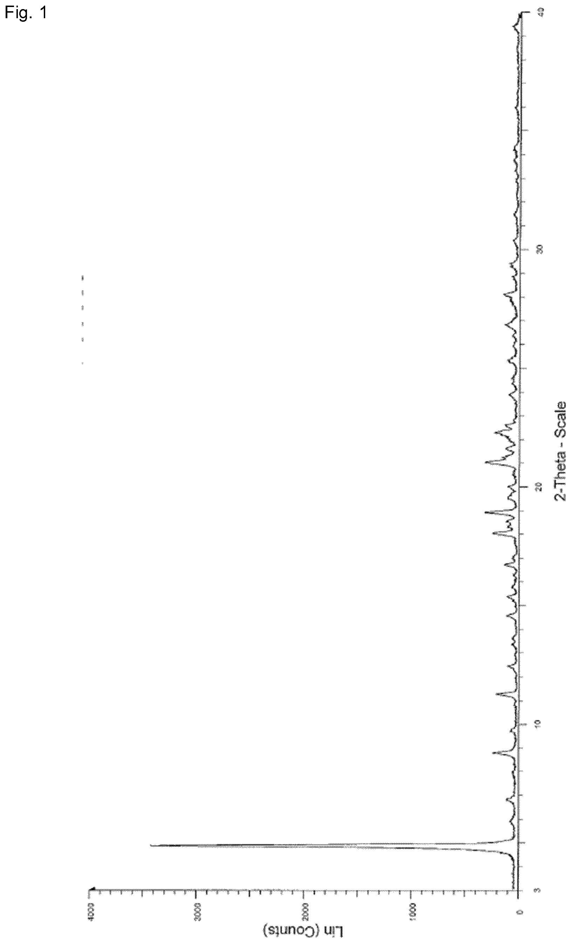

Especially, EP1294715 discloses, in the example 8, the preparation of N-{3-Chloro-4-[(3-fluorobenzyl)oxy]phenyl}-6-[5-({[2-(methanesulphonyl) ethyl]amino}methyl)-2-furyl]-4-quinazolinamine ditosylate anhydrate, i.e. Lapatinib ditosylate anhydrous, then it is converted, in example 10, to Lapatinib ditosylate monohydrate by means of treatment with pre-mixed 8:2 THF:deionized water solution at several temperatures, from 25°C to 80°C. The above mentioned process to obtain the said polymorph form of Lapatinb ditosylate anhydrous, having a specific powder x-ray diffraction pattern, disclosed in the example 9 and shown in Figure 1, it is carried out by means of tetrahydrofuran (THF) and an acid compound and produces 88% of yield. Said specific preparation, in particular the reaction with THF and an acid, could generate potentially genotoxic species, e.g. halo-alkyl derivatives that are unwanted in pharmaceutical products.

The publication WO2009/079541 discloses an another method for obtaining Lapatinb ditosylate anhydrous, that could be converted in Lapatinib ditosylate monohydrate. This method uses methanol and dimethylformamide, in particular in the Example 2, and produces a different polymorph form of Lapatinib ditosylate structure was collected on X-ray diffractometer, and in particular said crystal structure data was described in (d) part of the same example. Said polymorph is different from the polymorph form of example 8 of EP1294715. Furthermore anyone of the disclosed examples and the description of WO2009/079541 do not show the polymorph form of Lapatinb ditosylate anhydrous having the same powder X-ray diffraction pattern disclosed in EP1294715 in Figure 1.

Therefore, it appears that no one method disclosed in the examples of WO2009/079541 produces the same specific polymorph form disclosed in EP1294715.

Further several polymorphs of Lapatinib ditosylate have been described, US8252805 discloses many of them, in particular crystalline Form XIII of Lapatinib ditosylate is a polymorph, obtained using dimethylacetamide, in the example 19, and characterized by a PXRD pattern having peaks at about 5.9, 6.8, 8.9, 12.2, 13.6, 14.6, 16.0, 19.0, 20.4 and 22.9±0.2 degrees 2-theta, as illustrated in FIG. 23 in US8252805. Therefore the above-mentioned PXRD pattern does not show the same powder x-ray diffraction pattern disclosed in EP1294715, specifically in Figure 1.

The prior art methods, earlier described, suffer of the drawbacks that the productivity of Lapatinib ditosylate monohydrate is relatively low; furthermore the method of preparation of the above mentioned pharmaceutical active ingredient carried out using as starting material a disclosed polymorph form of Lapatinb ditosylate anhydrous, obtained by means of specific mixtures, in particular using tetrahydrofuran (THF) and an acid compound, could carry to the generation of potentially genotoxic species, not acceptable in a pharmaceutical active substance.

### Summary of invention

The problem addressed by the present invention is therefore that of providing an improved process for the preparation of Lapatinib ditosylate monohydrate, which allows to get round to the drawbacks above reported with reference to the known prior art, i.e. increase of the yield and/or no production of genotoxic impurities and/or reduction of the level of one or more specific impurities.

This problem is solved by a process for the preparation of Lapatinib ditosylate monohydrate as outlined in the annexed claims, whose definitions are integral part of the present description.

Particularly, the present invention provides a process for the preparation of Lapatinib ditosylate monohydrate of formula (I): 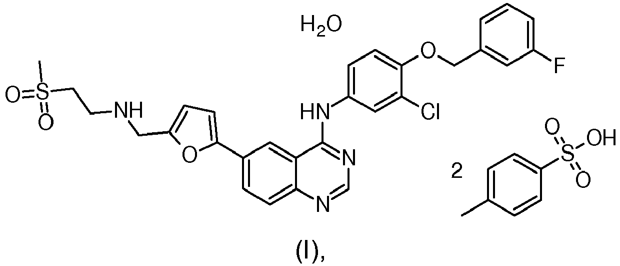 comprising the following steps:
a) crystallization or recrystallization of the starting material Lapatinib ditosylate anhydrous or an hydrated form thereof with a mixture of dimethylformamide and acetonitrile, wherein the volume of acetonitrile is double in comparison to the volume of dimethylformamide, to give solid Lapatinib ditosylate anhydrous of formula (II): 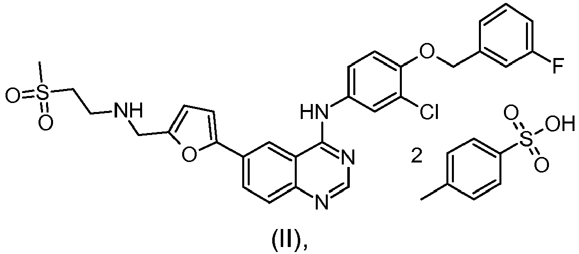 having X-ray powder diffraction pattern with characteristic peaks expressed in 2-Theta values (2θ) at: 4.8, 8.7, 11.2, 18.0, 18.9, 21.0, 22.3 each peak ±0.1;
b) conversion by hydration reaction of Lapatinib ditosylate anhydrous of formula (II) obtained in the step a) to Lapatinib ditosylate monohydrate of formula (I).

As another aspect, the process of the present invention provides a method for obtaining a decreased level of Fluorochloro aniline in the pharmaceutical active ingredient Lapatinib ditosylate monohydrate.

Further features and advantages of the process according to the invention will result from the description hereafter reported of examples of realization of the invention, provided as an indication and not as a limitation of the invention.

### Drawings

Fig. 1 shows the powder x-ray diffraction pattern of Lapatinib ditosylate anhydrous of formula (II), obtained by the step a) of the process of the present invention.
Fig. 2 shows the DSC curve of Lapatinib ditosylate anhydrous of formula (II) obtained by the step a) of the process of the present invention.
Fig. 3 shows the graph of filtration trends of the suspension of the compound of the step B) at three different pressures.

### Description of embodiments

Object of the present invention is a process for the preparation of Lapatinib ditosylate monohydrate compound of formula (I): 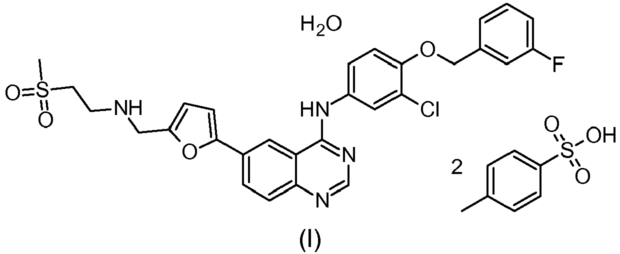 comprising the following steps:
a) crystallization or recrystallization of the starting material Lapatinib ditosylate anhydrous or an hydrated form thereof with a mixture of dimethylformamide and acetonitrile, wherein the volume of acetonitrile is double in comparison to the volume of dimethylformamide, to give solid Lapatinib ditosylate anhydrous of formula (II): 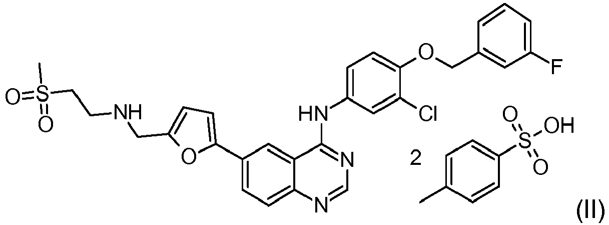 having X-ray powder diffraction pattern with characteristic peaks expressed in 2-Theta values (2θ) at: 4.8, 8.7, 11.2, 18.0, 18.9, 21.0, 22.3 each peak ±0.1;
b) conversion by hydration reaction of Lapatinib ditosylate anhydrous of formula (II) obtained in the step a) to Lapatinib ditosylate monohydrate of formula (I).

It has been indeed surprisingly found that the crystallization or recrystallization treatment, by a 1:2 (vol/vol) of dimethylformamide and acetonitrile, and the following hydration for obtaining Lapatinib ditosylate monohydrate of formula (I), allows an increase of the yield of pharmaceutical active ingredient, about from 90% to 95% of yield, and avoids the use of THF in presence of acid, i.e. the generation of potentially genotoxic species.

Another very important advantage of the process of the present invention is that said process is efficient in the reduction of the levels of the Fluorochloro aniline (abbreviated FCA), a significant genotoxic impurity.

Fluorochloro aniline (FCA) has the chemical name 3-chloro-4-[(3-fluorobenzyl)oxy]aniline, molecular formula C₁₃H₁₁CIFNO and has the following formula (V):

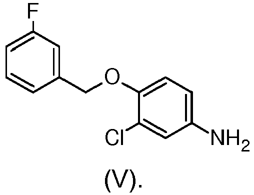

This substance is an essential intermediate in the process of the preparation of Lapatinib ditosylate monohydrate, and at the same time it is a genotoxic impurity that must be strongly reduced and avoided. The process of the present invention provides an efficient reduction, in particular by means of the crystallization or recrystallization treatment with a mixture of dimethylformamide and acetonitrile of the Lapatinib ditosylate anhydrous or an hydrate form, e.g. starting from an amount less 300 ppm of FCA, that is reduced at less of 12 ppm of FCA in the compound of formula (II) and/or of formula (I).

The further aspect of the process of the present invention is that, in this procedure, the step a) and the following step b) are carried out without discharging from the filter Lapatinib ditosylate anhydrous of formula (II) obtained in step a), therefore, starting from Lapatinib ditosylate anhydrous or an hydrated form thereof, thus avoiding the discharge, Lapatinib ditosylate monohydrate is obtained.

Finally the process of the present invention provides an higher yield than that disclosed in prior art, and a pharmaceutical active product having low levels of genotoxic impurities.

The starting material of the process of the present invention, Lapatinib ditosylate anhydrous or an hydrated form thereof, can be produced following one of more known synthesis routes.

Said starting material can be prepared, in particular starting from the method is disclosed in EP2754662 and proceeding according following synthesis scheme:

### Synthesis scheme.

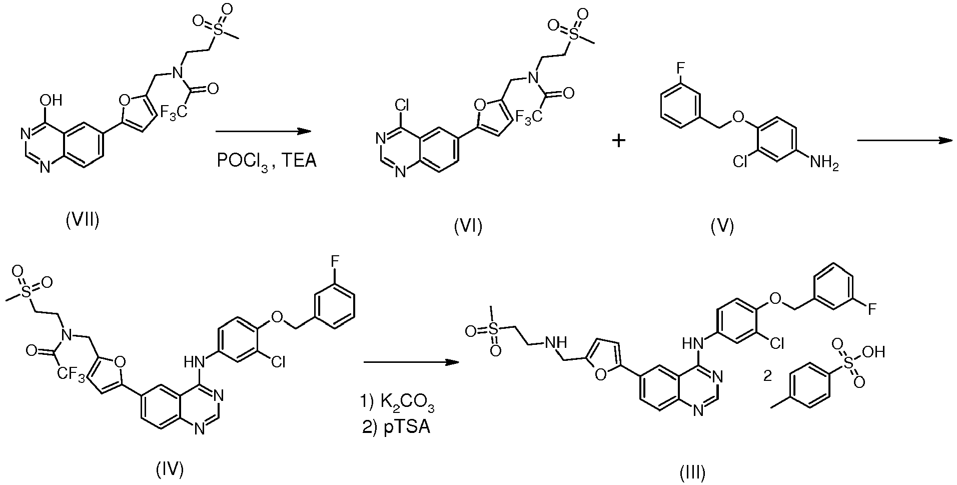

Following the disclosed process in said EP application, specifically in the examples from 1 to 6, the compound of formula (VII): 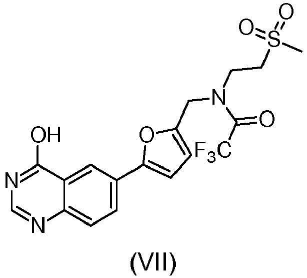 having the chemical name 2,2,2-trifluoro-N-{[5-(4-hydroxyquinazolin-6-yl)furan-2-yl]methyl}-N-[2-(methylsulfonyl)ethyl]acetamide can be prepared.

Then said compound is subjected a chlorination by means of Phosphorous oxychloride (POCl₃) and Triethylamine (TEA) to obtain the following compound of formula (VI): 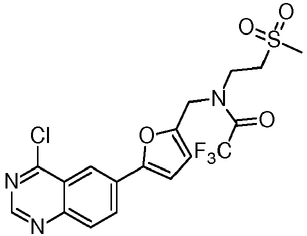 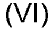 having the chemical name N-{[5-(4-chloroquinazolin-6-yl)furan-2-yl]methyl}-2,2,2-trifluoro-N-[2-(methylsulfonyl)ethyl]acetamide.

The subsequent reaction of the compound of formula (VI) with Fluorochloro aniline (FCA) of formula (V): 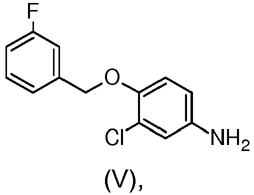 produces the compound of formula (IV): 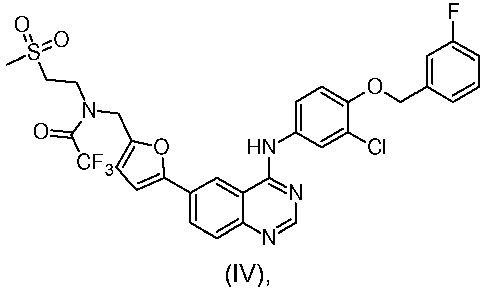 having the chemical name N-({5-[4-({3-chloro-4-[(3-fluorobenzyl)oxy] phenyl}amino)-6-quinazolinyl]-2-furyl}methyl)-2,2,2-trifluoro-N-[2-(methylsulfonyl)ethyl]acetamide.

The compound of formula (IV) reacting with aqueous potassium carbonate (K₂CO₃), produces Lapatinib free base, that after a crystallization in a mixture of dimethylformamide and acetonitrile by addition of p-toluenesulfonic acid (PTSA), isolation and drying, generates Lapatinib ditosylate of formula (III).

An other route of synthesis to obtain the starting material of the process of the present invention, is the method disclosed in US 7,157,466, in particular in the examples, wherein Lapatinib ditosylate anhydrous is produced.

According to the present invention, the starting material is Lapatinib ditosylate anhydrous or an hydrated form thereof, e.g. Lapatinib ditosylate monohydrate, dihydrate, trihydrate.

The term anhydrous means without any mole of water for 1 mole Lapatinib ditosylate.

The term hydrate means an amount of one mole of water for one mole of Lapatinib ditosylate, e.g. Lapatinib ditosylate monohydrate consists in 1 mole of water for 1 mole of Lapatinib ditosylate and Lapatinib ditosylate dihydrate consists in 2 mole of water for 1 mole of Lapatinib ditosylate.

According to the present invention, the starting material Lapatinib ditosylate anhydrous or an hydrated form thereof is a solid.

The process of the present invention is carried out by means of a crystallization or recrystallization of the starting material Lapatinib ditosylate anhydrous or an hydrated form.

The crystallization, in the present case, is a process of formation of solid crystals from a solution wherein the starting material, i.e. Lapatinib ditosylate anhydrous or an hydrated form thereof, it has been previously prepared and/or solubilized. In particular the term crystallization of the process of the present invention means either crystallization or recrystallization.

The recrystallization is a technique used for purifying chemical compounds and/or for obtaining a different solid forms (e.g. polymorphs), in particular a solid compound is solubilized in an appropriate solvent, and then the compound re-become a solid, typically a solid crystal, by means of heating and then cooling treatment or by solubilisation in a proper solvent and addition of an anti-solvent.

The process of the present invention can be thus, for example, carried out starting from a starting material and recrystallizing it by heating/cooling treatment or, alternatively, can be carried out by obtaining Lapatinib ditosylate anhydrous or an hydrated form thereof in solution and then crystallizing the product, for example, by cooling or by addition of an anti-solvent, or by other ways.

According to the process of the present invention, the crystallization or recrystallization of the starting material Lapatinib ditosylate anhydrous or an hydrated form thereof, in the step a), is carried out with a mixture dimethylformamide (DMF) and acetonitrile (ACN), to give solid Lapatinib ditosylate anhydrous of formula (II): 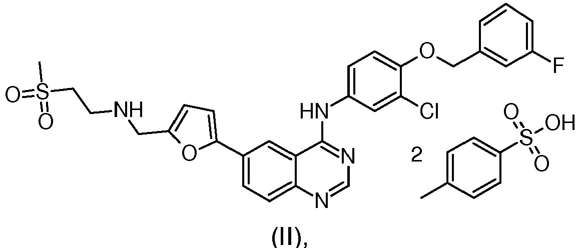 having the X-ray powder diffraction pattern with characteristic peaks expressed in 2-Theta values (2θ) at: 4.8, 8.7, 11.2, 18.0, 18.9, 21.0, 22.3, each peak ±0.1.

Dimethylformamide (DMF) and acetonitrile (ACN), beforehand mentioned, are used as solvents for give solid Lapatinib ditosylate anhydrous of formula (II), in particular acetonitrile is used as solvent and/or it has function of anti-solvent in the recrystallization; both solvents, DMF and ACN, guarantee a clean reaction, and provide a pure product of formula (II).

According to a preferred embodiment of the present invention, the compound of formula (II), obtained in step a), has the X-ray powder diffraction pattern with characteristic peaks expressed in 2-Theta values (2θ) at: 4.8, 6.8, 8.7, 11.2, 12.4, 14.5, 16.7, 18.0, 18.9, 19.5, 21.0, 22.3, 23.9, 25.3, 26.8, 28.1, 29.4, each peak ± 0.1.

According to an other preferred embodiment of the present invention, the compound of formula (II) obtained in step a), has melting point 246.14°C as measured by DSC.

According to a more preferred embodiment of the present invention, the compound of formula (II), obtained in step a), has X-ray powder diffraction pattern with characteristic peaks expressed in 2-Theta values (2θ) at: 4.8, 6.8, 8.7, 11.2, 12.4, 14.5, 16.7, 18.0, 18.9, 19.5, 21.0, 22.3, 23.9, 25.3, 26.8, 28.1, 29.4, each peak ± 0.1 and melting point 246.14°C as measured by DSC.

According to a preferred embodiment of the present invention, the step a) comprises the following steps:
A) adding dimethylformamide to Lapatinib ditosylate anhydrous or an hydrated form thereof and obtaining a solution of Lapatinib ditosylate anhydrous or an hydrated form thereof,
B) adding acetonitrile to the solution of the step A).

According the step A) the addition of dimethylformamide (DMF) solvent to Lapatinib ditosylate anhydrous or an hydrated form thereof is carried out for obtaining a solution of Lapatinib ditosylate anhydrous or an hydrated form thereof, specifically for obtaining a complete dissolution of Lapatinib ditosylate anhydrous or an hydrated in said solvent.

Solution means a solid compound dissolved in a solvent, for example the solution can be as an opalescent solution or a microsuspension or a suspension of little amount of solid material, typically an amount of insoluble material is less than 5% by weight of weight of the compound dissolved in a solvent. In the present invention the solution is obtained from the dissolution of Lapatinib ditosylate anhydrous or an hydrated form thereof solid form in dimethylformamide.

Specifically, in the present invention the compound in solution is completely dissolved, i.e. Lapatinib ditosylate anhydrous or an hydrated form thereof in dimethylformamide solvent produces a limpid solution, without the presence of any amount of solid material.

According to a preferred embodiment of the present invention, the step A) is carried out at a temperature comprised between 50°C and 80°C and/or the step B) is carried out at a temperature comprised between 50°C and 80°C.

In particular, the addition of dimethylformamide (DMF) in the step A) is carried out at a temperature comprised between 50°C and 80°C, specifically in a temperature comprised between 60°C and 80°C, for obtaining a complete dissolution of the starting material, Lapatinib ditosylate anhydrous or an hydrated form thereof, and the step B) is carried out at a temperature comprised between 50°C and 80°C, specifically at a temperature comprised between 50°C and 70°C, for obtaining a product containing a low amount of impurities, in particular an impurity having RRT 0.4, specifically said unknown impurity is reduced or removed by the means of the process of the present invention, as described in the example 6.

According to a preferred embodiment of the present invention, the addition of acetonitrile in step B) is carried out in a time period between 0.5 and 1 hour.

When the addition is carried out in said time period, a product with a lesser levels of impurities is obtained.

Said addition of acetonitrile in step B) is carried out in a time period between 0.5 and 1 hour by means of dripping said solvent into the solution of the step A).

Dripping means the addition of a amount of liquid substance is carried out slowly, drop by drop, spending time to add the all amount of above mention substance into a solution, i.e. the addition of acetonitrile to the solution of the step A).

According to a preferred embodiment of the present invention, step A) is carried out at a temperature comprised between 50°C and 80°C, specifically at a temperature comprised between 60°C and 80°, and/or step B) is carried out at a temperature comprised between 50°C and 80°C, specifically in a temperature comprised between 50°C and 70°C and the addition of acetonitrile in step B) is carried out in a time period between 0.5 and 1 hour.

According to the present invention, the volume of acetonitrile is double in comparison to the volume of dimethylformamide.

Volume means volume of solvent per unit of product being the weight of the starting material Lapatinib ditosylate anhydrous or an hydrated form thereof, which can be determined by stoichiometric calculations. Thus, for example, 1 volume is 1 Liter per 1 Kilo, or 1 mL for 1 gram, or 1 microliter per 1 milligram. In particular, in the present process the volume of acetonitrile is always double in comparison to the volume of dimethylformamide, e.g. 2 volumes of acetonitrile for 1 volume of dimethylformamide, or 3 volumes of acetonitrile for 1.5 volumes of dimethylformamide, or 4 volumes of acetonitrile for 2 volumes of dimethylformamide.

According to a more preferred embodiment of the present invention, step A) is carried out at a temperature comprised between 50°C and 80°C and/or step B) is carried out at a temperature comprised between 50°C and 80°C, the addition of acetonitrile in step B) is carried out in a time period between 0.5 and 1 hour and the volume of acetonitrile is double in comparison to the volume of dimethylformamide.

According to a preferred embodiment of the present invention, the ratio between the volume of dimethylformamide and acetonitrile are comprised respectively between 3.0:6.0 and 5.0:10.0 volumes, and the volume of acetonitrile is double in comparison to the volume of dimethylformamide.

According to a more preferred embodiment of the present invention, the above mentioned ratio between the volume of dimethylformamide and acetonitrile are comprised respectively between 3.0:6.0 and 5.0:10.0 volumes, and the volume of acetonitrile is double in comparison to the volume of dimethylformamide.

In particular, said ratio can be selected in the group comprising the following ratios: 3.0:6.0, 3.1:6.2, 3.2:6.4, 3.3:6.6, 3.4:6.8, 3.5:7.0, 3.6:7.2, 3.7:7.4, 3.8:7.6, 3.9:7.8, 4.0:8.0, 4.1:8.2, 4.2:8.4, 4.3:8.6, 4.4:8.8, 4.5:9.0, 4.6:9.2,4.7:9.4,4.8:9.6,4.9:9.8,5.0:10.0.

The ratio means the comparison of relative sizes of two or more values. Thus, in the present process, one of more ratio between the volume of dimethylformamide (DMF) and the volume of acetonitrile (ACN) is 3.0:6.0, i.e. 3.0 volumes of DMF and 6.0 volumes of ACN, specifically both volumes are referred to the amount of starting material Lapatinib ditosylate anhydrous or an hydrated form thereof used to carry out the invention.

According to a more preferred embodiment of the present invention, the ratio between the volume of dimethylformamide and acetonitrile is respectively 4.5:9.0.

Another embodiment of the present invention, the ratio between the volume of dimethylformamide and acetonitrile is respectively 4.5:9.0 ±10%. In particular, according the ratio above described, the volume of dimethylformamide is 4.5 ±10% and the volume of acetonitrile 9 ±10%, both volumes are referred to the amount of starting material Lapatinib ditosylate anhydrous or an hydrated form thereof.

According to a preferred embodiment of the present invention, the filtration of the suspension of compound of formula (II) of the step B) is carried out at pressure between 2 and 4 bar.

Suspension means an amount of solid material suspended in a solution, i.e. the compound of formula (II) of the step B) is suspended in the solution consisting in the mixture of dimethylformamide and acetonitrile.

Said filtration is carried out washing with acetonitrile the suspension of compound of formula (II) of the step B), the latter obtained by means of upon cooling.

Said filtration of the suspension of compound of the step B) is carried out at pressure between 2 and 4 bar, because in said range the filtration and filtration time are better than the filtration carried out at pressure value which is more then 4 bar or less then 2 bar, probably due to a compression phenomenon of the cake.

These values of pressure were tested to be optimum in the large filtration, in fact following the filtration method with said pressure, shorter filtration time and larger amount filtered are achieved.

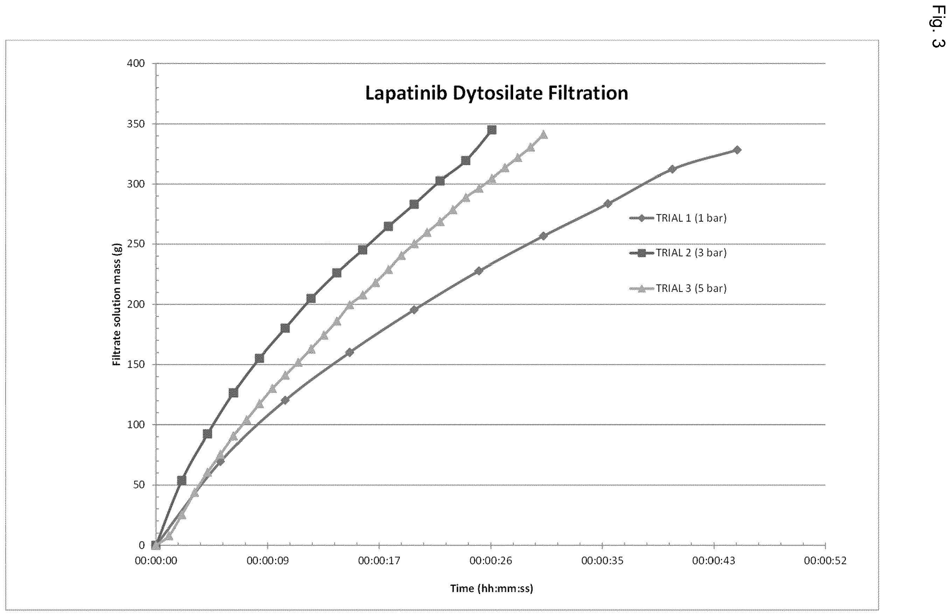

In particular data of filtered solution mass versus time were collected for three trials, with three values of pressure, said data are reported in example 5 and plotted in the graph of the trends of figure 3.

According to a preferred embodiment of the present invention, the conversion by hydration reaction of step b) is carried out at a temperature comprised between 50°C and 70°C.

In particular, said conversion of step b) by hydration reaction of Lapatinib ditosylate anhydrous of formula (II) obtained in the step a) to produce Lapatinib ditosylate monohydrate of formula (I) is carried out by means of only water, preferably purified water.

According to a preferred embodiment of the present invention, the step a) and the following step b) are carried out without discharging from the filter Lapatinib ditosylate anhydrous of formula (II) obtained in step a).

Specifically, the step a) and the step b) can be carried out without discharging the solid of Lapatinib ditosylate anhydrous of formula (II) obtained in step a); i.e. starting from step a) and continuing to step b), thus producing a solid Lapatinib ditosylate monohydrate of formula (I), keeping the compound of Lapatinib ditosylate anhydrous of formula (II) obtained in step a) in the filter, i.e. without discharging it from the filter.

According to a more preferred embodiment of the present invention, in the step a) the crystallization or recrystallization is carried out with the ratio of volume of dimethylformamide and acetonitrile respectively 4.5:9.0, and the filtration of the suspension of compound of formula (II) of the step B) is carried out at pressure 3 bar and in the step b) the conversion by hydration reaction is carried out at a temperature comprised between 50°C and 55°C.

One of the many important results obtained from the process of the present invention, further to an increase of the yield of Lapatinib ditosylate monohydrate of formula (I), about from 90% to 95% of yield, is a reduction the levels of the Fluorochloro aniline (FCA), a significant genotoxic impurity, as described beforehand.

In particular by means of the crystallization or recrystallization treatment of Lapatinib ditosylate anhydrous or hydrate with a mixture of dimethylformamide and acetonitrile, e.g. starting from an amount less 300 ppm of FCA, that is reduced at less of 12 ppm of FCA (see examples 1, 2 and 6) in the compound of formula (II) and/or of formula (I).

Another aspect of the invention is thus, the process for the preparation of Lapatinib ditosylate anhydrous of formula (II): 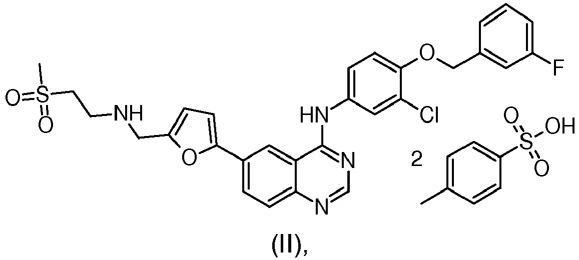 having X-ray powder diffraction pattern with characteristic peaks expressed in 2-Theta values (2θ) at: 4.8, 8.7, 11.2, 18.0, 18.9, 21.0, 22.3, each peak ± 0.1; or with following characteristic peaks expressed in 2-Theta values (2θ) at: 4.8, 6.8, 8.7, 11.2, 12.4, 14.5, 16.7, 18.0, 18.9, 19.5, 21.0, 22.3, 23.9, 25.3, 26.8, 28.1, 29.4, each peak ± 0.1, and melting point 246.14°C, is carried out by means of crystallization or recrystallization of starting material Lapatinib ditosylate anhydrous or an hydrated form thereof with a mixture of dimethylformamide and acetonitrile.

According to a preferred embodiment of the present invention, the conditions, beforehand described, for obtaining Lapatinib ditosylate anhydrous of formula (II) are applied.

In particular, the process for the preparation of Lapatinib ditosylate anhydrous of formula (II) is carried out following step a), which comprises the steps A) and B) beforehand above mentioned.

The temperatures, the volumes, the ratio earlier described concerning the step A) are the same used for obtaining a solution of Lapatinib ditosylate anhydrous or an hydrated form thereof.

The temperatures, time period of addition of acetonitrile and the filtration pressure earlier described concerning of the step B) are the same realised to prepare Lapatinib ditosylate anhydrous of formula (II).

### EXPERIMENTAL SECTION

Compounds are named using the basic rules of IUPAC Nomenclature.

X-ray Powder Diffraction (XRPD) analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 0.0205° per second.

Differential scanning calorimetry (DSC) analysis was recorded with a Mettler Toledo DSC1. The system is equipped with Full Range Sensor (FRS5), the sensor having 56 thermocouples and provides high sensitivity.

The following table lists the abbreviations used:
- T: Temperature
- RT: Retention time
- Tₘₐₓ: Maximum Temperature
- °C: Degree Centigrade or Degree Celsius
- V: Volume
- M: minutes
- g: Gramme
- mL: Millilitre
- A: Area
- Vs: Versus
- MW: Molecular weight
- THF: Tetrahydrofuran
- DMF: Dimethylformamide
- ACN: Acetonitrile
- POCl₃: Phosphorous oxychloride
- K₂CO₃: Potassium carbonate
- NaOH: Sodium hydroxide
- TEA: Triethylamine
- FCA: Fluorocloro anilina
- DSC: Differential scanning calorimetry
- XPRD: X-ray Powder Diffraction
- NMR: Nuclear magnetic resonance
- HPLC: High performance liquid chromatography

The starting material Lapatinib ditosylate anhydrous or an hydrated form
thereof can be prepared according to known prior art methods, or for example, as described in EP2754662 or can be purchased on the market.

### Example 1: Preparation of Lapatinib ditosylate of formula (III), as starting material of the present invention.

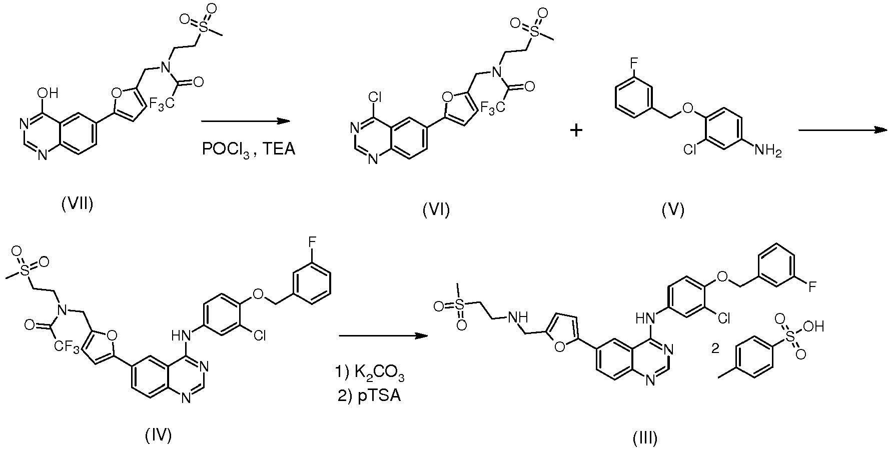

The compound of formula (VII) was prepared according to the method disclosed in EP2754662, in particular in the experimental part from example 1 to 6.

A glass reactor was charged 100 g of the compound of formula (VII) and 833 ml of ACN, under nitrogen atmosphere. After was added 69.2 ml of POCl₃ at 20-25°C and was washed the dosing line with 83 mL of ACN. Then was added 47.9 g of TEA and the dosing line was washed with 83 mL of ACN. At the end of dosing the reaction mixture was stirred at T=80-85°C for at least 6 h and the compound of formula (VI) was produced. The obtained residue, after the reaction, was taken up with 500 mL of toluene and was evaporated in vacuo to residue at Tₘₐₓ jacket 80°C, the beforehand described process was repeat for two times. Later the residue was taken up with 665 mL of ACN and was warmed at T=80-85°C,at least 2 h a solution of 50.22 g of FCA (154 ppm)and 335 ml of ACN were added, the obtained reaction mixture was warmed at T=80-85°C and was stirred at least 1 h. After the reaction the mixture was cooled at T= 50-60°C and a solution of 187 g of K₂CO₃ and 500 mL of water were charged, the all was warmed at T=65°C for 11 h and compound of formula (IV) was produced, but it wasn't isolated. Then the reaction mixture was cooled at T=20-25°C and the pH was corrected at 12-12.5 with 13 ml of NaOH 30% solution.

The mixture was stirred for 10 M , then aqueous layer was separated. The obtained mixture was evaporated in vacuo to residue at Tₘₐₓ jacket 60°C.

The residue was taken up with a solution of 230 mL of DMF and 150 mL of ACN and was stirred at T=65-75°C. After a solution of p-Toluensulfonic acid monohydrate (MW = 190.22), ACN and DMF were charged in about 1 h to reaction mixture (optionally PTSA was added) and the obtained mixture was stirred at T=20-25°C for 1 h and then was cooled in about 1 h to T=0-5°C. The mixture was filtered washing with a solution of 200 mL of ACN and 100 mL of DMF and after draining the washing was repeated wit the same solution. Finally the cake (0.24% of RRT 0.4 impurity) was dried in vacuo at 20-25 °C for at least 6 h and then at max 70 °C (Jacket T) for at least 6 h and 159.2 g of Lapatinib ditosylate of formula (III), having 25 ppm of FCA and 0.20% of the unknown impurity with RRT 0.4, were obtained (76,50% molar yield).

### Example 2: Preparation of Lapatinib ditosylate anhydrous of formula (II), as described in the step a). Evidence of the invention.

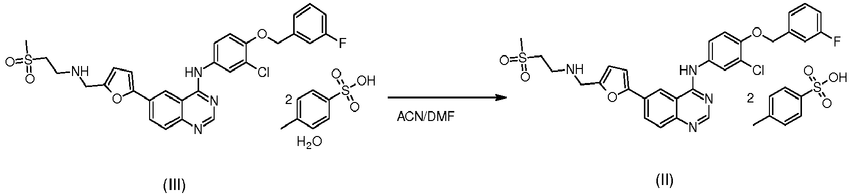

A glass reactor was charged 150 g of Lapatinib ditosylate of formula (III), (25 ppm of FCA and 0.20% of RRT 0.4 impurity), obtained in the example 1, and 375 g of DMF (2.5 V of compound (III)) under nitrogen atmosphere. The reaction mixture was warmed at T=70-80°C until complete dissolution, after that 75 mL of DMF (1/2 V of compound (III)) was added and the all was warmed at T=70-80°C. Then 900 g of ACN (6 V of compound (III)) was dripped for 0.5 h, and the reaction mixture was warmed at T=70-80°C and stirred. Then the mixture was cooled to T=20-25°C and was stirred for 1 h and again it was cooled to T=0-5°C and was stirred for 2 h. The obtained mixture was filtered washing with 2X150 mL of pre-cooled (T=0-5°C) ACN (2 V of compound (III)). Finally the cake was dried in vacuo at 20-25 °C for at least 6 h and then at max 70 °C (Jacket T) for at least 6 h and 144.4 g of Lapatinib ditosylate anhydrous of formula (II), having 5 ppm of FCA and less then 0.05% of the unknown impurity with RRT 0.4, were obtained.

In the above described example the volumes of CAN are double of the volumes of DMF, i.e. respectively 6.0:3.0.

### Example 3: Characterization of Lapatinib ditosylate anhydrous of formula (II) by XPRD. Shown in Figure 1.

XRPD analysis was performed using a Bruker AXS D8 Advance diffractometer with monochromated CuKα radiation in Bragg-Brentano geometry. The system is equipped with a scintillator detector. The diffractogram was recorded from 3° to 40° (2θ) at a scan rate of 3 sec/step and scan step of 0.02°.

Lapatinib ditosylate anhydrous of formula (II) prepared in (as prepared i.e. in example 2) was analyzed and the XRPD diffractogram, aquired before and after a gentle milling treatment is shown in Fig. 1. In particular, Lapatinib ditosylate anhydrous of formula (II) has a characteristic X-ray powder diffraction pattern with characteristic peaks expressed in 2-Theta values (2θ) at: 4.8, 8.7, 11.2, 18.0, 18.9, 21.0, 22.3, each peak ±0.1.

More in particular, Lapatinib ditosylate anhydrous of formula (II) has a characteristic X-ray powder diffraction pattern with characteristic peaks expressed in 2-Theta values (2θ) at: 4.8, 6.8, 8.7, 11.2, 12.4, 14.5, 16.7, 18.0, 18.9, 19.5, 21.0, 22.3, 23.9, 25.3, 26.8, 28.1, 29.4, each peak ± 0.1.

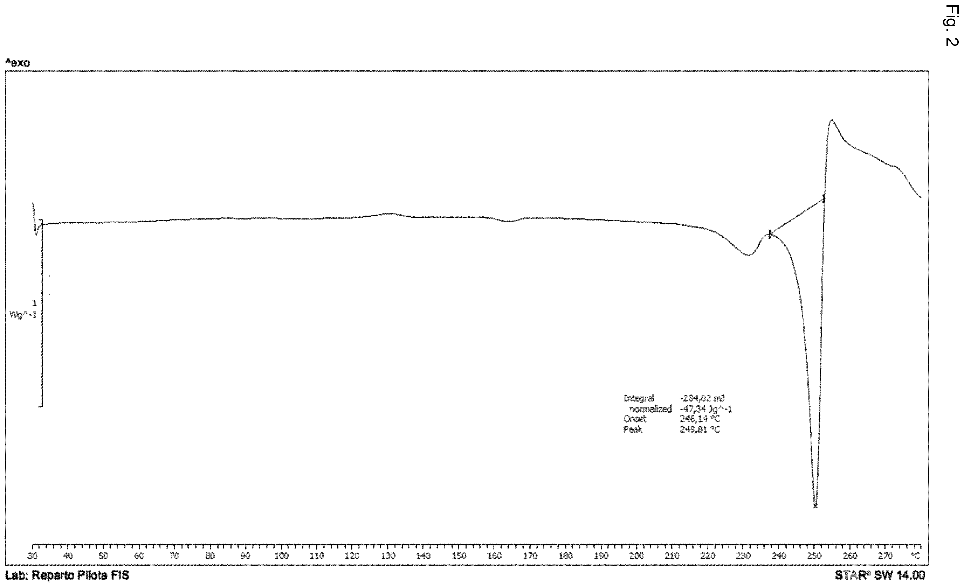

### Example 4: Characterization of Lapatinib ditosylate anhydrous of formula (II) by DSC. Shown in Figure 2.

DSC analysis was recorded with a Mettler Mettler Toledo DSC1. A sample of 2.00-5.00 mg was weighed into a 40 µL aluminium crucible with a pinhole lid and was heated, under nitrogen (80 mL/min), at 10 °C/min from 30 to 280 °C.

The solid form of Lapatinib ditosylate anhydrous of formula (II) is characterized by an endothermic peak corresponding to the melting point with an onset at 246.14°C (fusion enthalpy -47.34 J/g), measured by DSC analysis (10°C/min). At 249.81°C (onset) begin a broad endotermic peak.

### Example 5: The filtration trends of the suspension of the compound of the step B) at three different pressures. Shown in Figure 3.

The slurry containing the product to isolate was divided in three equal parts to perform three filtration trials at different pressure. The following tables summarize the parameters of each filtration trials.

| GENERAL FILTRATION PARAMETERS | |
|---|---|
| Batch size | 150 g of crude Lapatinib ditosylate |
| Filtration media (µm) | 20 |
| Filtration area (m2) | 0.00196 |
| Filtration temperature (°C) | 5 |
| Solid real density (kg/m3) | 1820 |
| Solvent density (kg/m3) | 872 |

| TRIAL PARAMETERS | TRIAL 1 | TRIAL 2 | TRIAL 3 |
|---|---|---|---|
| Differential pressure (bar) | 1 | 3 | 5 |
| Slurry charged (g) | 399.9 | 406.3 | 433 |
| Dry filtered solid (g) | 43.2 | 39.9 | 51.2 |
| Cake height (mm) | 30 | 30 | 30 |

Data of filtered solution mass versus time was collected for each trial. Said filtration trends graph is shown in Figure 3.

As reported in the graph, the 5 bar trial showed a lower filtration rate than the 3 bar trial. This was probably due to a compression phenomenon of the cake. According to these results, the optimum filtration is carried-out at a pressure below 3 bar.

### Example 6: Preparation of Lapatinib ditosylate monohydrate of formula (I), as described in the step b). Effect of the invention.

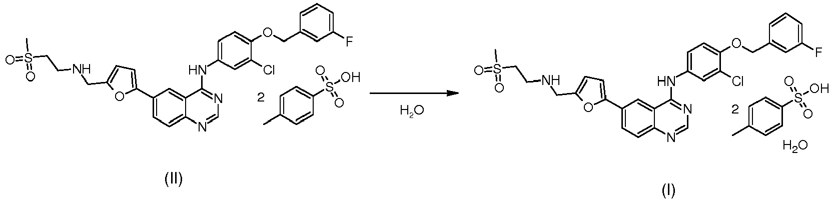

A glass reactor was charged 144.4 g of Lapatinib ditosylate anhydrous of formula (II), obtained in the example 2, and 1500 mL of water and the all was stirred at T= 50-55°C for 24 h; during that time, i.e. during the crystalline form transition was added 500 mL of water. Then the slurry was cooled at T=20-25°C and was stirred for 1 h and was filtered, on Hastelloy cloth, washing with 2X450 mL of water.

Finally the cake was dried in vacuo at 20-25 °C for 6 h and then at max 55 °C 6 h and 136 g of Lapatinib ditosylate monohydrate of formula (I), having 5 ppm of FCA and less then 0.05% of the unknown impurity with RRT 0.4, were obtained (90.18% yield).

### Example 7: Preparation of Lapatinib ditosylate monohydrate of formula (I), without discharging from the filter Lapatinib ditosylate anhydrous of formula (II) obtained in step a).

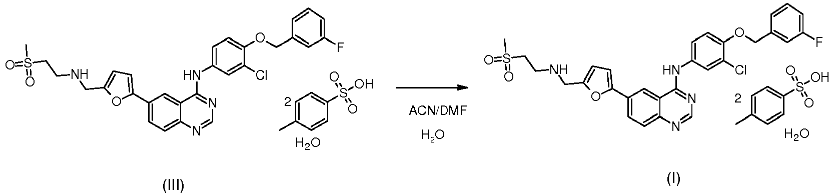

A glass reactor was charged 150 g of Lapatinib ditosylate of formula (III), (25 ppm of FCA and 0.20% of RRT 0.4 impurity), and 375 g of DMF (2.5 V of compound (III)) under nitrogen atmosphere. The reaction mixture was warmed at T=70-80°C until complete dissolution, after that 75 mL of DMF (1/2 V of compound (III)) was added and the all was warmed at T=70-80°C. Then 900 g of ACN (6 V of compound (III)) was dripped for 0.5 h, and the reaction mixture was warmed at T=70-80°C and stirred. Then the mixture was cooled to T=20-25°C and was stirred for 1 h and again it was cooled to T=0-5°C and was stirred for 2 h. The obtained mixture was filtered washing with 2X150 mL of pre-cooled (T=0-5°C) ACN (2 V of compound (III)). The cake was dried in vacuo at 20-25 °C for at least 6 h and then at max 70 °C (Jacket T) for at least 6 h and 144.4 g of Lapatinib ditosylate anhydrous of formula (II), having 5 ppm of FCA and less then 0.05% of the unknown impurity with RRT 0.4, were obtained. 144.4 g of the obtained compound of formula (II), without discharging the same from the filter, were collected/picked up, in a glass reactor, with 1500 mL of water, and the all was stirred at T= 50-55°C for 24 h; during that time, i.e. during the crystalline form transition was added 500 mL of water. Then the slurry was cooled at T=20-25°C and was stirred for 1 h and was filtered, on Hastelloy cloth, washing with 2X450 mL of water.

Finally the cake was dried in vacuo at 20-25 °C for 6 h and then at max 55°C 6 h and 138 g of Lapatinib ditosylate monohydrate of formula (I), having 5 ppm of FCA and less then 0.05% of the unknown impurity with RRT 0.4, were obtained (92% yield).

### Example 8: Analytic method for determining the amount of impurities of the present invention.

### Method A: The unknown impurity, i.e. RRT 0.4, could be identified and monitored via the following HPLC method:

### Chromatographic conditions:

| | | | |
|---|---|---|---|
| Column: | Poroshell 120 PFP 100 x 4.6, 4 µm | | |
| Temp. Column: | 45°C | | |
| Column back pressure: | 210 bar (initial conditions) | | |
| Mobile Phase A: | Ammonium acetate 50 mM, pH 4.0 | | |
| Mobile Phase B: | Methanol | | |

| Gradient: | Time (min) | %A | %B |
|---|---|---|---|
| | 0 | 80 | 20 |
| | 10 | 35 | 65 |
| | 35 | 10 | 90 |
| | 37.1 | 80 | 20 |
| Post run: | 5 min | | |
| Flow: | 1.5 mL/min | | |
| Detector: | UV 268 nm | | |
| Injection Volume: | 5 µL | | |
| Temp. Autosampler: | 25°C | | |
| Analysis Time: | 35 min | | |
| Sample diluent: | 50:50 Water:Acetonitrile | | |

RRT 0.4 means relative retention time, referred to the elution time of Lapatinib.

### Method B1: Fluorochloro aniline (FCA) impurity, could be identified and monitored via the following HPLC method, when the amount is less 300 ppm of FCA :

| | | | |
|---|---|---|---|
| Column: | Kinetex Phenyl Hexyl, 100 x 4.6, 5 µm | | |
| Temp. Column: | 40°C | | |
| Mobile Phase A: | mixture water/methanol (9:1, volume/volume) containing 0.1 % ammonium acetate | | |
| Mobile Phase B: | mixture water/methanol (1:9, volume/volume) containing 0.1 % ammonium acetate | | |

| Gradient: | Time (min) | %A | %B |
|---|---|---|---|
| | 0 | 70 | 30 |
| | 2 | 70 | 30 |
| | 20 | 0 | 100 |
| | 23 | 0 | 100 |
| | 23.1 | 70 | 30 |
| Post run: | 5 min | | |
| Flow: | 2.0 mL/min | | |
| Detector: | UV 210 nm | | |
| Injection Volume: | 5 µL | | |
| Temp. Autosampler: | 5°C | | |
| Analysis Time: | 23 min | | |
| Sample diluent: | Methanol | | |
| Diluent | Water:Acetonitrile 50:50 | | |

### Method B2: Fluorochloro aniline (FCA) impurity, could be identified and monitored via the following LC-MS method, when the amount is less 12 ppm of FCA :

| | | | |
|---|---|---|---|
| Column: | Kinetex XB C18 100A, 50 x 2.1 mm, 1.7 µm | | |
| Temp. Column: | 45°C | | |
| Mobile Phase A: | Water : Acetonitrile : Formic Acid 950 : 50 : 1 v : v : v (for LC-MS) | | |
| Mobile Phase B: | Acetonitrile (for LC-MS) | | |

| Gradient: | Time (min) | %A | %B |
|---|---|---|---|
| | 0 | 100 | 0 |
| | 1.50 | 5 | 95 |
| | 1.90 | 2 | 98 |
| | 2.50 | 2 | 98 |
| | 2.60 | 100 | 0 |
| Flow: | 0.700 mL/min | | |
| Detector: | UV, 210 nm (not used for quantification, only for detect the matrix peaks) | | |
| Injection Volume: | 0.5 µL | | |
| Temp. Autosampler: | 15 ± 4°C | | |
| MS Tune Page (Waters Xevo TQ-S) | Ion Mode: ES+ | | |
| | Capillary: 1.0 kV | | |
| | Cone: 30.0 V | | |
| | Source Offset: 50 V | | |
| | Source Temperature: 150°C | | |
| | Desolvation Temperature: 600 °C | | |
| | Desolvation gas flow: 1000 L/h | | |
| MRM Method (Waters Xevo TQ-S) | Acquisition Time Range: Start at 0 min, End at 2.5 min | | |
| Transition for | Transition: 252.10 Da -> 143.00 Da | | |
| quantification | Dwell: 0.025 s | | |
| | Collision: 20 eV | | |
| Transition for | Transition: 252.10 Da -> 109.10 Da | | |
| identification | Dwell: 0.025 s | | |
| | Collision: 20 eV | | |
| Diluent: | Water: Acetonitrile 50 : 50 | | |

## Claims

1. A process for the preparation of Lapatinib ditosylate monohydrate of formula (I): 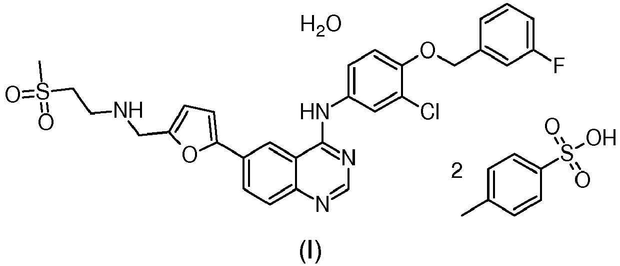 comprising the following steps:
a) crystallization or recrystallization of the starting material Lapatinib ditosylate anhydrous or an hydrated form thereof with a mixture of dimethylformamide and acetonitrile, wherein the volume of acetonitrile is double in comparison to the volume of dimethylformamide, to give solid Lapatinib ditosylate anhydrous of formula (II): 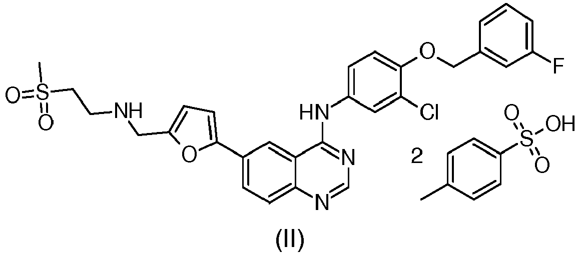 having X-ray powder diffraction pattern with characteristic peaks expressed in 2-Theta values (2θ) at: 4.8, 8.7, 11.2, 18.0, 18.9, 21.0, 22.3, each peak ± 0.1;
b) conversion by hydration reaction of Lapatinib ditosylate anhydrous of formula (II) obtained in the step a) to Lapatinib ditosylate monohydrate of formula (I).

2. Process according to the claim 1, wherein the compound of formula (II) has X-ray powder diffraction pattern with characteristic peaks expressed in 2-Theta values (2θ) at: 4.8, 6.8, 8.7, 11.2, 12.4, 14.5, 16.7, 18.0, 18.9, 19.5, 21.0, 22.3, 23.9, 25.3, 26.8, 28.1, 29.4, each peak ± 0.1.

3. Process according to anyone of the claims from 1 to 2, wherein the compound of formula (II) has melting point of 246.14°C as measured by DSC.

4. Process according to the claim 1, wherein the step a) comprises the following steps:
A) adding dimethylformamide to Lapatinib ditosylate anhydrous or an hydrated form thereof and obtaining a solution of Lapatinib ditosylate anhydrous or an hydrated form thereof,
B) adding acetonitrile to the solution of the step A).

5. Process according to the claim 4, wherein the step A) is carried out at a temperature comprised between 50°C and 80°C and/or the step B) is carried out at a temperature comprised between 50°C and 80°C.

6. Process according to the claim 4, wherein the addition of acetonitrile in step B) is carried out in a time period comprised between 0.5 and 1 hour.

7. Process according to anyone claims from 4 to 6, wherein the step A) is carried out at a temperature comprised between 50°C and 80°C and/or step B) is carried out at a temperature comprised between 50°C and 80°C, the addition of acetonitrile in step B) is carried out in a time period between 0.5 and 1 hour and the volume of acetonitrile is double in comparison to the volume of dimethylformamide.

8. Process according to anyone claims from 1 to 7, wherein the ratio between the volume of dimethylformamide and acetonitrile are comprised respectively between 3.0:6.0 and 5.0:10.0 volumes.

9. Process according to anyone claims from 1 to 8, wherein the ratio between the volume of dimethylformamide and acetonitrile are respectively 4.5:9.0.

10. Process according to anyone claims from 4 to 9, wherein the filtration of the suspension of compound of the step B) is carried out at pressure between 2 and 4 bar.

11. Process according to anyone claims from 1 to 10, wherein the conversion by hydration reaction of step b) is carried out at a temperature comprised between 50°C and 70°C.

12. Process according to anyone claims from 1 to 11, wherein the step a) and the following step b) are carried out without discharging from the filter Lapatinib ditosylate anhydrous of formula (II) obtained in step a).

13. Process according to anyone claims from 4 to 12, wherein in the step a) the crystallization or recrystallization is carried out with the ratio of volume of dimethylformamide and acetonitrile of 4.5:9.0 and the filtration of the suspension of compound of formula (II) obtained in the step B) is carried out at pressure 3 bar and in the step b) the conversion by hydration reaction is carried out at a temperature comprised between 50°C and 55°C.

14. Process for the preparation of Lapatinib ditosylate anhydrous of formula (II): 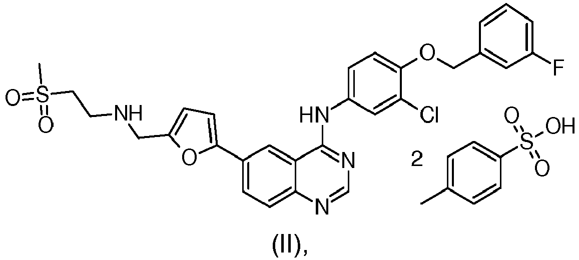 having X-ray powder diffraction pattern as described in claim 1 or claim 2, and having melting point according to claim 3, carried out by means of crystallization or recrystallization of starting material Lapatinib ditosylate anhydrous or an hydrated form thereof with a mixture of dimethylformamide and acetonitrile, wherein the volume of acetonitrile is double in comparison to the volume of dimethylformamide.

15. Process according to the claim 14, wherein the conditions of anyone of the claims from 4 to 10 are applied.

## Patentansprüche

1. Verfahren zur Herstellung von Lapatinibditosylat-Monohydrat mit der Formel (I): 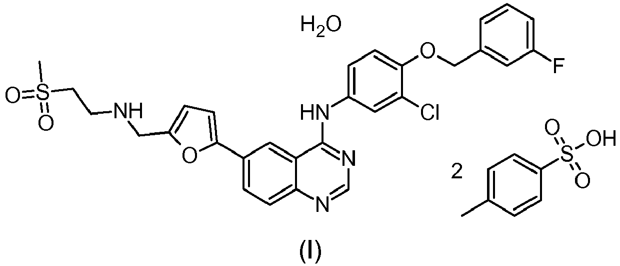 umfassend die folgenden Schritte:
a) Kristallisierung oder Umkristallisierung des Ausgangsmaterials Lapatinibditosylat-wasserfrei oder in einer hydratisierten Form davon mit einer Mischung aus Dimethylformamid und Acetonitril, wobei das Volumen des Acetonitrils im Vergleich zu dem Volumen des Dimethylformamids das Doppelte ist, um festes Lapatinibditosylat-wasserfrei mit der Formel (II) zu ergeben: 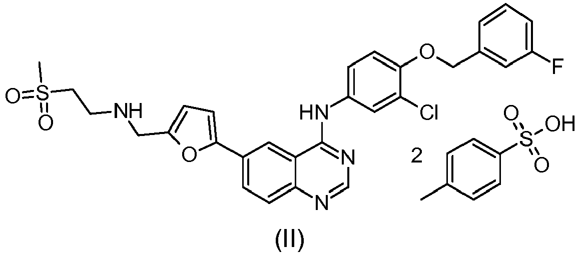 mit einem Pulverröntgenbeugungsspektrum mit charakteristischen Peaks, angegeben in Werten für 2 Theta (2θ), bei: 4,8, 8,7, 11,2, 18,0, 18,9, 21,0, 22,3, jeder Peak ± 0,1;
b) Konvertierung des in Schritt a) erhaltenen Lapatinibditosylats-wasserfrei mit der Formel (II) durch Hydratisierungsreaktion zu Lapatinibditosylat-Monohydrat mit der Formel (I).

2. Verfahren nach Anspruch 1, wobei die Verbindung mit der Formel (II) das folgende Pulverröntgenbeugungsspektrum mit charakteristischen Peaks, ausgedrückt in Werten von 2 Theta (2θ), bei: 4,8, 6,8, 8,7, 11,2, 12,4, 14,5, 16,7, 18,0, 18,9, 19,5, 21,0, 22,3, 23,9, 25,3, 26,8, 28,1, 29,4 hat, jeder Peak ± 0,1.

3. Verfahren nach einem der Ansprüche 1 bis 2, wobei die Verbindung mit der Formel (II) einen Schmelzpunkt von 246,14 °C aufweist, gemessen mittels DSC.

4. Verfahren nach Anspruch 1, wobei der Schritt a) die folgenden Schritte umfasst:
A) Zufügen von Dimethylformamid zu Lapatinibditosylat-wasserfrei oder einer hydratisierten Form davon, und Erhalten einer Lösung von Lapatinibditosylat-wasserfrei oder einer hydratisierten Form davon,
B) Zufügen von Acetonitril zu der Lösung aus Schritt A).

5. Verfahren nach Anspruch 4, wobei Schritt A) bei einer Temperatur zwischen 50 °C und 80 °C durchgeführt wird und/oder der Schritt B) bei einer Temperatur zwischen 50 °C und 80 °C durchgeführt wird.

6. Verfahren nach Anspruch 4, wobei die Zugabe von Acetonitril in Schritt B) in einem Zeitraum durchgeführt wird, der zwischen 0,5 und 1 Stunde liegt.

7. Verfahren nach einem der Ansprüche 4 bis 6, wobei der Schritt A) bei einer Temperatur zwischen 50 °C und 80 °C durchgeführt wird und/oder Schritt B) bei einer Temperatur zwischen 50 °C und 80 °C durchgeführt wird, die Zugabe von Acetonitril in Schritt B) in einem Zeitraum zwischen 0,5 und 1 Stunde durchgeführt wird und das Volumen an Acetonitril im Vergleich zu dem Volumen an Dimethylformamid das Doppelte beträgt.

8. Verfahren nach einem der Ansprüche 1 bis 7, wobei das Verhältnis zwischen dem Volumen an Dimethylformamid und Acetonitril jeweils zwischen 3,0:6,0 und 5,0:10,0 Volumina liegt.

9. Verfahren nach einem der Ansprüche 1 bis 8, wobei das Verhältnis zwischen dem Volumen an Dimethylformamid und Acetonitril jeweils 4,5:9,0 beträgt.

10. Verfahren nach einem der Ansprüche 4 bis 9, wobei die Filtration der Suspension der Verbindung von Schritt B) bei einem Druck zwischen 2 und 4 bar durchgeführt wird.

11. Verfahren nach einem der Ansprüche 1 bis 10, wobei die Konvertierung durch Hydratisierungsreaktion von Schritt b) bei einer Temperatur zwischen 50 °C und 70 °C durchgeführt wird.

12. Verfahren nach einem der Ansprüche 1 bis 11, wobei der Schritt a) und der folgende Schritt b) durchgeführt werden, ohne das in Schritt a) erhaltene Lapatinibditosylat-wasserfrei mit der Formel (II) aus dem Filter auszutragen.

13. Verfahren nach einem der Ansprüche 4 bis 12, wobei in Schritt a) die Kristallisation oder Umkristallisation mit dem Verhältnis des Volumens an Dimethylformamid und Acetonitril von 4,5:9,0 durchgeführt wird und die Filtration der Suspension der Verbindung mit der Formel (II), die in Schritt B) erhalten wurde, mit einem Druck von 3 bar durchgeführt wird, und in Schritt b) die Konvertierung durch Hydratisierungsreaktion mit einer Temperatur zwischen 50 °C und 55 °C durchgeführt wird.

14. Verfahren zur Herstellung von Lapatinibditosylat-wasserfrei mit der Formel (II): 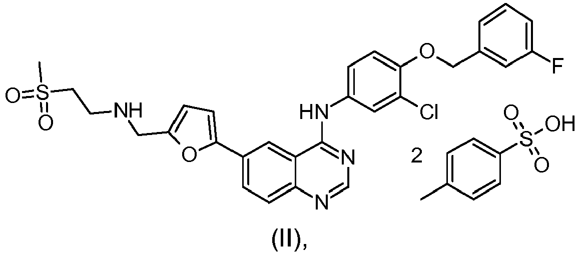 mit einem Pulverröntgenbeugungsspektrum wie in Anspruch 1 oder Anspruch 2 beschrieben, und mit einem Schmelzpunkt gemäß Anspruch 3, das mittels Kristallisation oder Umkristallisation des Ausgangsmaterials Lapatinibditosylat-wasserfrei oder einer hydratisierten Form davon mit einer Mischung von Dimethylformamid und Acetonitril durchgeführt wird, wobei das Volumen an Acetonitril im Vergleich zu dem Volumen an Dimethylformamid das Doppelte ist.

15. Verfahren nach Anspruch 14, wobei die Bedingungen gemäß einem der Ansprüche von 4 bis 10 angewendet werden.

## Revendications

1. Procédé pour la préparation de ditosylate de Lapatinib monohydraté de formule (I): 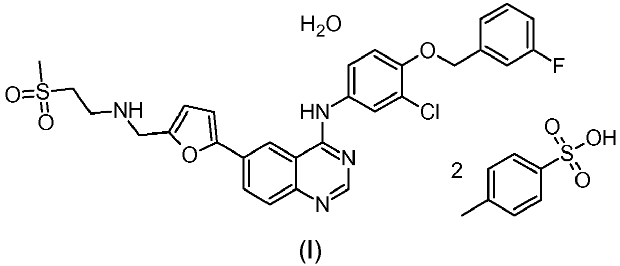 comprenant les étapes suivantes :
a) cristallisation ou recristallisation de la matière de départ ditosylate de Lapatinib anhydre ou d'une forme hydratée de celui-ci avec un mélange de diméthylformamide et d'acétonitrile, le volume d'acétonitrile étant double par rapport au volume de diméthylformamide, pour donner du ditosylate de Lapatinib solide de formule (II): 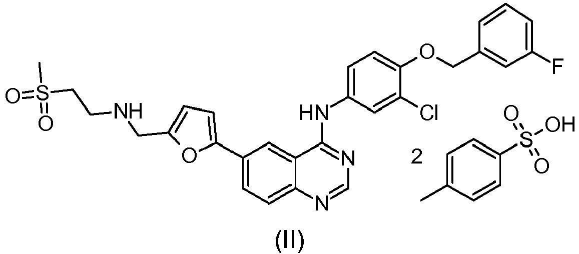 ayant un motif de diffraction de poudre de rayons X avec des pics caractéristiques exprimés en valeurs 2-Thêta (2θ) à : 4,8, 8,7, 11,2, 18,0, 18,9, 21,0, 22,3, chaque pic ± 0,1 ;
b) conversion par réaction d'hydratation du ditosylate de Lapatinib anhydre de formule (II) obtenu à l'étape a) en ditosylate de Lapatinib monohydraté de formule (I).

2. Procédé selon la revendication 1, dans lequel le composé de formule (II) a un motif de diffraction de poudre de rayons X avec des pics caractéristiques exprimés en valeurs 2-Thêta (2θ) à : 4,8, 6,8, 8,7, 11,2, 12,4, 12,4, 14,5, 16,7, 18,0, 18,9, 19,5, 21,0, 22,3, 23,9, 25,3, 26,8, 28,1, 29,4, chaque pic ± 0,1.

3. Procédé selon l'une quelconque des revendications 1 et 2, le composé de formule (II) ayant un point de fusion de 246,14 °C tel que mesuré par DSC.

4. Procédé selon la revendication 1, l'étape a) comprenant les étapes suivantes :
A) ajout de diméthylformamide à du ditosylate de Lapatinib anhydre ou une forme hydratée de celui-ci et obtention d'une solution de ditosylate de Lapatinib anhydre ou d'une forme hydratée de celui-ci,
B) ajout d'acétonitrile à la solution de l'étape A).

5. Procédé selon la revendication 4, l'étape A) étant réalisée à une température comprise entre 50 °C et 80 °C et/ou l'étape B) étant réalisée à une température comprise entre 50 °C et 80 °C.

6. Procédé selon la revendication 4, l'ajout d'acétonitrile à l'étape B) étant réalisé pendant une durée comprise entre 0,5 et 1 heure.

7. Procédé selon l'une quelconque des revendications 4 à 6, l'étape A) étant réalisée à une température comprise entre 50 °C et 80 °C et/ou l'étape B) étant réalisée à une température comprise entre 50 °C et 80 °C, l'ajout d'acétonitrile à l'étape B) étant réalisé pendant une durée comprise entre 0,5 et 1 heure et le volume d'acétonitrile étant double par rapport au volume de diméthylformamide.

8. Procédé selon l'une quelconque des revendications 1 à 7, le rapport entre le volume de diméthylformamide et d'acétonitrile étant compris entre des volumes de respectivement 3,0:6,0 et 5,0:10,0.

9. Procédé selon l'une quelconque des revendications 1 à 8, le rapport entre le volume de diméthylformamide et d'acétonitrile étant respectivement de 4,5:9,0.

10. Procédé selon l'une quelconque des revendications 4 à 9, la filtration de la suspension du composé de l'étape B) étant réalisée à une pression entre 2 et 4 bar.

11. Procédé selon l'une quelconque des revendications 1 à 10, la conversion par réaction d'hydratation de l'étape b) étant réalisée à une température comprise entre 50 °C et 70 °C.

12. Procédé selon l'une quelconque des revendications 1 à 11, l'étape a) et l'étape b) suivante étant réalisées sans déchargement à partir du filtre du ditosylate de Lapatinib anhydre de formule (II) obtenu à l'étape a).

13. Procédé selon l'une quelconque des revendications 4 à 12, la cristallisation ou la recristallisation à l'étape a) étant réalisée avec le rapport de volume de diméthylformamide et d'acétonitrile de 4,5:9,0 et la filtration de la suspension du composé de formule (II) obtenue à l'étape B) étant réalisée à une pression 3 bar et la conversion par réaction d'hydratation à l'étape b) étant réalisée à une température comprise entre 50 °C et 55 °C.

14. Procédé pour la préparation de ditosylate de Lapatinib anhydre de formule (II): 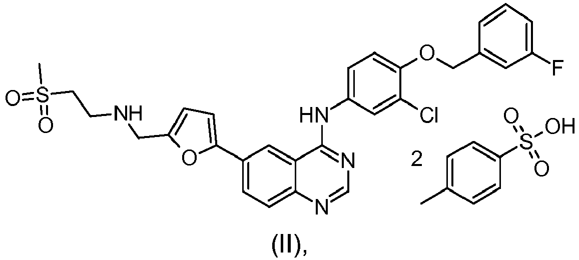 ayant un motif de diffraction de poudre de rayons X tel que décrit dans la revendication 1 ou la revendication 2, et ayant un point de fusion selon la revendication 3, réalisé par cristallisation ou recristallisation de la matière de départ ditosylate de Lapatinib anhydre ou d'une forme hydratée de celui-ci avec un mélange de diméthylformamide et d'acétonitrile, le volume d'acétonitrile étant double par rapport au volume de diméthylformamide.

15. Procédé selon la revendication 14, les conditions de l'une quelconque des revendications 4 à 10 étant appliquées.
